# EUROPEAN PATENT SPECIFICATION

(11) **EP 3 636 322 B1**
(45) Date of publication and mention of the grant of the patent: **28.07.2021**
(21) Application number: 18199772.7
(22) Date of filing: 11.10.2018
(51) Int. Cl.: A61Q 5/06, A61K 8/25, A61K 8/73, A61Q 11/00, A61K 8/11, A61K 8/22, A61K 8/87, A61K 8/86, A61K 8/55, A61K 8/58, A61K 8/41, A61K 8/19

(54) **STABILIZED HYDROGEN PEROXIDE COMPOSITION**
STABILISIERTE WASSERSTOFFPEROXIDZUSAMMENSETZUNG
COMPOSITION DE PEROXYDE D'HYDROGÈNE STABILISÉE

(43) Date of publication of application: 15.04.2020
(73) Proprietor: Cavex Holland BV, 2031 CJ Haarlem (NL)
(72) Inventor: Spaid, Matthew, 1018DZ Amsterdam (NL); Woortman, Richard, 1991NR Velserbroek (NL); Helversteijn, Peter, 2182MA Hillegom (NL); den Boer, Hans Maarten, 1852XH Heiloo (NL)
(74) Representative: Bendele, Tanja

(56) References cited:
- ES-T3- 2 350 245
- US-A- 5 059 417
- US-A1- 2002 141 949
- US-A1- 2006 009 371
- US-A1- 2014 147 402
- US-A1- 2016 106 641
- US-A1- 2016 346 179

## Description

Disclosed is a stabilized aqueous hydrogen peroxide composition comprising a copolymer of an ammonium or alkali salt of acryloyldimethyltaurate and vinylpyrrolidone for encapsulating a mixture comprising at least hydrogen peroxide, water and poly(ethylene oxide) homopolymer, or for forming a matrix comprising a mixture of at least hydrogen peroxide, water and poly(ethylene oxide) homopolymer.

The composition comprising 2 wt. % to 30 wt. % of hydrogen peroxide, an amount of water, 1 wt. % to 10 wt. % poly(ethylene oxide)-poly(propylene oxide) block copolymer, 0,01 wt. % to 10 wt. % of a salt of a comprising a copolymer of an ammonium or alkali salt of acryloyldimethyltaurate and vinylpyrrolidone and 30 wt. % to 95 wt. % poly(ethylene oxide) homopolymer, 0,01 wt. % to 5 wt. % stabilizer system comprising 0,1 to 2 wt. % and 0,01 wt. % to 1 wt. % of a chelating agent or a mixture of chelating agents, wherein the composition comprises in total 100 wt. %.

Many bleaching compositions are known. US7341636B2 discloses an alkaline hydrogen peroxide composition comprising aromatic chelating agents and a pH above 7.0. US2016/106641A1 discloses 2-acrylamido-2-methylpropane sulfonic acids polymers or copolymers in compositions. US2016/0346179A1 relates to a dual oral composition with a peroxide-containing component comprising poloxamer 407. Further peroxide containing compositions are disclosed in US2006/0009371A1, US5059417, US2002/0141949A1 and ES2350245T3. US 2014/0147402A1 disclosed the use of hydroxypyridones a polymer with thickening properties for peroxide containing compositions. The viscosity of these compositions change within four weeks with or without hydroxypyridone significant. In general, hydrogen peroxide is acceptable for use in bleaching or whitening compositions with regard to the decomposition products water and oxygen. However, the unintended decomposition of hydrogen peroxide due to contaminations of substances with traces of metal ions makes the utilization of such applications very difficult. For acidic compositions with a pH less than 5.0 stabilizers such as sodium stannate, phosphorus compounds or other chelating agents are known.

Object of the invention was to provide a composition with a less acidic pH, in particular in the range of above 5.0 and less than 7.0, wherein the composition shall be stable towards decomposition of hydrogen peroxide, in particular at room temperature. Further the composition should release the peroxide quickly. In addition, the composition should have a variable viscosity to enable the preparation of compositions to be applied by e.g. syringe, pen, bite splint or single-dose. One particular object was to provide a composition that is stable at room temperature. Moreover, it was an object of the invention to provide a composition which remains on the tooth surface with a high penetration potential.

According to the invention the object is solved with a composition according to claim 1 and the use according to claim 13.

According to the invention a composition is disclosed, wherein the composition comprises
i 2 wt. % to 30 wt. % of hydrogen peroxide
ii water, in particular 2 wt. % to 30 wt. % water,
iii 1 wt. % to 10 wt. % poly(ethylene oxide)-poly(propylene oxide) block copolymer
iv 0,01 wt. % to 10 wt. % of a salt of a co-polymer comprising copolymer of an ammonium or alkali salt of acryloyldimethyltaurate and vinylpyrrolidone,
v 30 wt. % to 95 wt. % poly(ethylene oxide) homopolymer, in particular 35 wt. % to 85 wt. % of poly(ethylene oxide) homopolymer,
vi 0,01 wt. % to 5 wt. % stabilizer system, in particular 0,1 to 2 wt. %, wherein the stabilizer system is selected from a) phosphorus containing stabilizer or mixture of phosphorus containing stabilizers selected from the group consisting of pyrophosphates, in particular diethylene triamine penta(methylene phosphonic acid), hexamethylene diamine tetra(methylene phosphonic acid), bis hexamethylene triamine pentamethylene phosphonic acid and etidronic acid, and b) stannate stabilizer, and/or c) alkali hydroxide, base and/or buffers,
vii 0,01 wt. % to 1 wt. % of a chelating agent or a mixture of chelating agents, wherein the composition comprises in total 100 wt. %.

The composition can be used as an oral care application, in particular as a tooth bleaching composition/tooth whitening composition. The viscosity of the composition may be adapted to easily enable the application via syringe, pen or single-dose application.

A preferred composition has reversible temperature-sensitive properties; in particular, the composition is a thermo-sensitive gel. Further, in a preferred embodiment the composition of the present invention possesses a sole-state at room temperature and a gel-state at a physiological temperature, the latter being in the range of about 35 to 38 °C or around 37 °C.

According to the invention compound iv is a copolymer of an ammonium or alkali salt of acryloyldimethyltaurate and vinylpyrrolidone, in particular an ammonium salt, most preferred is an ammonium salt of copolymer of acryloyldimethyltaurate and vinylpyrrolidone (Acrylamido methyl propane sulfonic acid polymer).

A preferred compound iv of the above-mentioned polymer or co-polymer has a molecular weight in the range from 10.000 to 450.000 g/mol, in particular from 100.000 g/mol to 350.000 g/mol, more preferred from 150.000 to 275.000 g/mol. Most preferred is a molecular weight in the range of 225.000 g/mol +/- 50.000 g/mol. Further, it is preferred when the compound iv the polymer or co-polymer has a viscosity in the range from 45000 to 67000 mPas (1 w.-% in dist. H₂O).

Preferred compounds iv may comprise Ammonium Acryloyldimethyltaurate/VP Copolymer, Sodium Acryloyldimethyl Taurate/Acrylamide/VP Copolymer, and/or Sodium Acryloyldimethyltaurate/VP Crosspolyme.

The composition according to the invention comprises in a preferred embodiment from 0,5 wt. % to 6 wt. %, more preferred 0.5 wt. % to 5 wt. %, of a salt, in particular of an ammonium or alkali salt, of a copolymer of acryloyldimethyltaurate and vinylpyrrolidone, preferred is an ammonium salt of a copolymer of acryloyldimethyltaurate and vinylpyrrolidone.

The used poly(ethylene oxide)-poly(propylene oxide) block copolymer, in particular poly(ethylene oxide)-poly(propylene oxide)- poly(ethylene oxide) block forms micelles and/or functions as emulsifier and controlling agent to adjust the viscosity of the composition. Typically molecular weights of the poly(ethylene oxide)-poly(propylene oxide) block copolymer, in particular poly(ethylene oxide)-poly(propylene oxide)- poly(ethylene oxide) block, may range from 1.000 g/mol to 15.000 g/mol, preferably from 2.000 g/mol to 15.000 g/mol, most preferred is from 1.000 to 5.000 g/mol.

The poly(ethylene oxide)-poly(propylene oxide) block copolymer is used as a thermo-reversible gelling agent. In particular the poly(ethylene oxide)-poly(propylene oxide) block copolymer and the poly(ethylene oxide) homopolymer are used in combination to provide a thermo-adapting gel composition, in particular a thermo-reversible thermoplastic gel. In particular the poly(ethylene oxide)-poly(propylene oxide) block copolymer and the poly(ethylene oxide) homopolymer mixture swells in the mouth at the temperature of a human being.

The poly(ethylene oxide) homopolymer forms a mixture with hydrogen peroxide and water. Preferred poly(ethylene oxide) homopolymers have a low molar mass of below 10.000 g/mol. Further a preferred poly(ethylene oxide) homopolymer comprises terminal hydroxyl groups. A particular preferred poly(ethylene oxide) homopolymer has a molar mass of 100 to 5.000 g/mol, more preferred form 100 to below 500 g/mol. The most preferred poly(ethylene oxide) homopolymer has a molar mass of 150 to 250 g/mol, e.g. PEG 200 (molar mass 190-210 g/mol).

A particular preferred composition comprises
i 5 wt. % to 15 wt. % of hydrogen peroxide,
ii 5 wt. % to 15 wt. % of water,
iii 2 wt. % to 10 wt. % poly(ethylene oxide)-poly(propylene oxide) block copolymer,
iv 0,01 wt. % to 10 wt. % salt of a co-polymer comprising a co-polymer of an ammonium or alkali salt of acryloyldimethyltaurate and vinylpyrrolidone,
v 60 wt. % to 85 wt. % poly(ethylene oxide) homopolymer, in particular with terminal hydroxy groups, particularly preferred 65 wt. % to 85 wt. % of poly(ethylene oxide) homopolymer,
vi 0,01 wt. % to 5 wt. % stabilizer system selected from a) phosphorous containing stabilizer or mixture of phosphorus containing stabilizers selected from the group consisting of pyrophosphates, and b) stannate stabilizer, and/or c) alkali hydroxide, bae and/or buffers, in particular 0,1 to 2 wt. %,
vii 0,01 wt. % to 1 wt. % of a chelating agent or a mixture of chelating agents, wherein the composition comprises in total 100 wt. %. In a preferred embodiment the content of i hydrogen peroxide and ii water is equal.

An alternative preferred composition comprises
i 15 wt. % to 30 wt. % of hydrogen peroxide,
ii 15 wt. % to 30 wt. % of water,
iii 2 wt. % to 10 wt. % poly(ethylene oxide)-poly(propylene oxide) block copolymer,
iv 0,01 wt. % to 10 wt. % an ammonium or alkali salt of a copolymer of acryloyldimethyltaurate and vinylpyrrolidone,
v 30 wt. % to 60 wt. % poly(ethylene oxide) homopolymer, in particular with terminal hydroxy groups, particularly preferred 35 wt. % to 50 wt. % of poly(ethylene oxide) homopolymer,
vi 0,01 wt. % to 5 wt. % stabilizer system selected from a) phosphorous containing stabilizer or mixture of phosphorus containing stabilizers selected from the group consisting of pyrophosphates, and b) stannate stabilizer, and/or c) alkali hydroxide, bae and/or buffers, in particular 0,1 to 2 wt. %,
vii 0,01 wt. % to 1 wt. % of a chelating agent or a mixture of chelating agents,
wherein the composition comprises in total 100 wt. %. In a preferred embodiment the content of i hydrogen peroxide and ii water is equal.

In one embodiment the composition may comprise a weight content (wt. %) of hydrogen peroxide and water in the range from 2 : 1 to 1 : 2, in particular 1:1 +/- 10 %.

As compositions comprising aqueous hydrogen peroxides are typically acidic, it may be necessary to add a base to the composition, such as aqueous potassium hydroxide or aqueous sodium hydroxide until the desired pH is attained. Aqueous potassium hydroxide is preferred. The base or alkali should be free from metal ions that would catalyse the decomposition of the hydrogen peroxide, such as ferrous ions, ferric ions, cupric ions, cuprous ions, manganous ions, and similar transition metal ions. The base or alkali should also be free from both organic and inorganic materials that would react with the hydrogen peroxide. The stabilized composition comprising aqueous hydrogen peroxide typically comprises about 1 wt. % to about 30 wt. %, typically about 4 wt. % to about 20 wt. %, more typically about 4 wt. % to about 12 wt. %, even more typically about 5 wt. % to about 10 wt. %, of hydrogen peroxide. Further it is preferred that the components i to v, in particular the hydrogen peroxide and all polymers, each comprise less than 10 ppm-wt. % metal ions.

In one embodiment, component vii, the chelating agent or the mixture of chelating agents, is selected from ethylenediaminetetraacetic acid, alkali and/or alkali earth salt of ethylenediaminetetraacetic acid (EDTA), aromatic chelating agents and a mixture thereof, in particular alkali and/or alkali earth salt of ethylenediaminetetraacetic acid (EDTA), particularly preferred are potassium and/or sodium EDTA. Preferred aromatic chelating agents may be selected from salts of salicylic acid, substituted salicyclic acid, 6-hydroxy-piconilic acid, substituted 6-hydroxy-picolinic acid, 8-hydroxy-quinoline, and substituted 8-hydroxy-quinolines.

In a further embodiment in the vi stabilizer system the preferred c) alkali hydroxides are potassium hydroxide and/or sodium hydroxide. A particular preferred vi stabilizer system is selected from a) etidronic acid (1-hydroxyethane-1,1-diphosphonic acid) and b) stannate stabilizer, in particular sodium stannate trihydrate.

A preferred poly(ethylene oxide)-poly(propylene oxide) block copolymer, in particular a poly(ethylene oxide)-poly(propylene oxide)-poly(ethylene oxide) block is selected from poly(ethylene oxide)-poly(propylene oxide)-poly(ethylene oxide) block copolymers with a medium molecular weight of 9840 to 14600 Dalton (Dalton D = unit for the relative molecular mass). The hydroxy value of the block copolymer may be from 44.0 to 55.0 (KOH/g % w/w). In addition, the pH of the block copolymer may be in the range of 5.0 to 7.5 (2.5 % in water).

The pH of a preferred composition is higher than 5.0, in particular the pH is in the range of 5.0 to 7.5, and particular preferred is a pH in the range of 5.1 to 6.5.

The viscosity of the composition may vary from 1 to 1000 Pa·s, preferred is a range from 5 to 200 Pa·s (ISO28399).

A further embodiment of the invention is the use of a salt of a co-polymer comprising a copolymer of an ammonium or alkali salt of acryloyldimethyltaurate and vinylpyrrolidone for encapsulation of a mixture comprising at least hydrogen peroxide, water and poly(ethylene oxide) homopolymer, or for forming a matrix comprising a mixture of at least hydrogen peroxide, water and poly(ethylene oxide) homopolymer.

It will be apparent to those skilled in the art, that in aqueous neutral, acidic or basic solution one or more of the above-mentioned components may be present as its corresponding anion or anions, or as an equilibrium mixture of the component and its corresponding anion or anions. Anions of these components and mixtures of these components and their corresponding anions are included in the definition of each of these components and are within the scope of the claims.

The advantageous properties of this invention are shown by the following examples.

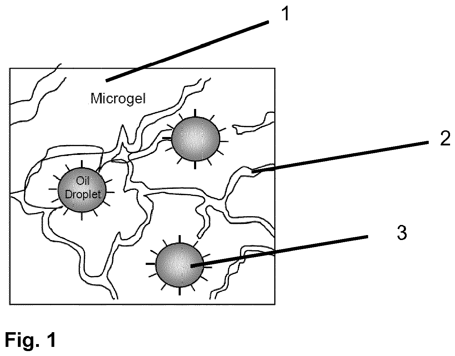

A schematically illustration of the microgel 1 and the encapsulation by the ammonium salt of the co-polymer of acrylic acid and vinylpyrrolidone 2 of the micelles 3 forming the oil droplets is shown in figure 1.

**Table 1: Examples 1 to 3**

| **compound** | **belongs to** | **example 1 wt. %** | **example 2 wt. %** | **example 3 wt. %** |
|---|---|---|---|---|
| PEG 200 | v | 40 | 72 | 78 |
| H₂O₂ | i | 25 | 9 | 6 |
| water | ii | 25 | 9 | 6 |
| Poloxamer | iii | 5 | 5 | 5 |
| Aristoflex | iv | 1 | 1 | 1 |
| KOH | vi | < 1 | < 1 | < 1 |
| Etidronic acid | vi | < 1 | < 1 | < 1 |
| EDTA (potassium) | vii | < 1 | < 1 | < 1 |
| Sodium stannate trihydrate | vi | < 1 | < 1 | < 1 |

**Table 2: Examples 4 to 6**

| **compound** | **belongs to** | **example 4 wt. %** | **example 5 wt. %** | **example 6 wt. %** |
|---|---|---|---|---|
| PEG 200 | v | 40 | 72 | 78 |
| H₂O₂ | i | 25 | 9 | 6 |
| water | ii | 25 | 9 | 6 |
| Poloxamer | iii | 5 | 5 | 5 |
| Aristoflex | iv | 4 | 4 | 4 |
| KOH | vi | < 1 | < 1 | < 1 |
| Etidronic acid | vi | < 1 | < 1 | < 1 |
| EDTA (potassium) | vii | < 1 | < 1 | < 1 |
| Sodium stannate trihydrate | vi | < 1 | < 1 | < 1 |

The viscosity of these compositions is in the range of 10 to 100 Pa·s (ISO28399).

## Claims

1. A composition comprising
i 2 wt. % to 30 wt. % of hydrogen peroxide,
ii water,
iii 1 wt. % to 10 wt. % poly(ethylene oxide)-poly(propylene oxide) block copolymer,
iv 0,01 wt. % to 10 wt. % of a salt of a co-polymer comprising a copolymer of an ammonium or alkali salt of acryloyldimethyltaurate and vinylpyrrolidone,
v 30 wt. % to 95 wt. % poly(ethylene oxide) homopolymer,
vi 0,01 wt. % to 5 wt. % stabilizer system selected from
a) phosphorus containing stabilizer or mixture of phosphorus containing stabilizers selected from the group consisting of pyrophosphates, and
b) stannate stabilizer, and/or c) alkali hydroxide, base and/or buffers, and
vii 0,01 wt. % to 1 wt. % of a chelating agent or a mixture of chelating agents, wherein the composition comprises in total 100 wt. %.

2. Composition according to claim 1, comprising
i 5 wt. % to 15 wt. % of hydrogen peroxide,
ii 5 wt. % to 15 wt. % of water,
iii 2 wt. % to 10 wt. % poly(ethylene oxide)-poly(propylene oxide) block copolymer,
iv 0,01 wt. % to 10 wt. % salt of a co-polymer comprising a co-polymer of an ammonium or alkali salt of acryloyldimethyltaurate and vinylpyrrolidone,
v 60 wt. % to 85 wt. % poly(ethylene oxide) homopolymer,
vi 0,01 wt. % to 5 wt. % stabilizer system selected from a) phosphorus containing stabilizers or mixture of phosphorus containing stabilizer selected from the group consisting of pyrophosphates, and b) stannate stabilizer, and/or c) alkali hydroxide, base and/or buffers, and
vii 0,01 wt. % to 1 wt. % of a chelating agent or a mixture of chelating agents, wherein the composition comprises in total 100 wt. %.

3. Composition according to claim 1, comprising
i 15 wt. % to 30 wt. % of hydrogen peroxide,
ii 15 wt. % to 30 wt. % of water,
iii 2 wt. % to 10 wt. % poly(ethylene oxide)-poly(propylene oxide) block copolymer,
iv 0,01 wt. % to 10 wt. % salt of a co-polymer comprising a copolymer of an ammonium or alkali salt of acryloyldimethyltaurate and vinylpyrrolidone,
v 30 wt. % to 60 wt. % poly(ethylene oxide) homopolymer,
vi 0,01 wt. % to 5 wt. % stabilizer system selected from a) phosphorus containing stabilizer or mixture of phosphorus containing stabilizers selected from the group consisting of pyrophosphates, and b) stannate stabilizer, and/or c) alkali hydroxide, base and/or buffers, and
vii 0,01 wt. % to 1 wt. % of a chelating agent or a mixture of chelating agents,
wherein the composition comprises in total 100 wt. %.

4. Composition according to any of claims 1 to 3, wherein the weight content (wt. %) of hydrogen peroxide and water are from 2 : 1 to 1 : 2, in particular 1 : 1 +/- 10 %.

5. Composition according to any of claims 1 to 4, wherein
iv the co-polymer is selected from a copolymer of an ammonium of acryloyldimethyltaurate and vinylpyrrolidone.

6. Composition according to any of claims 1 to 3, wherein
vii the chelating agent or the mixture of chelating agents is selected from ethylenediaminetetraacetic acid, alkali and/or alkali earth salt of ethylenediaminetetraacetic acid (EDTA), aromatic chelating agent and a mixture thereof, in particular alkali and/or alkali earth salt of ethylenediaminetetraacetic acid (EDTA), particular preferred are potassium and/or sodium EDTA.

7. Composition according to any of claims 1 to 3, wherein
vi pyrophosphate is selected from diethylene triamine penta(methylene phosphonic acid), hexamethylene diamine tetra(methylene phosphonic acid), bis (hexamethylene triamine penta(methylene phosphonic acid)) and etidronic acid.

8. Composition according to any of claims 1 to 3, wherein
vi the b) stannate stabilizer is sodium stannate trihydrate.

9. Composition according to any of claims 1 to 7, wherein the composition comprises v 35 wt. % to 85 wt. % of poly(ethylene oxide) homopolymer.

10. Composition according to any of claims 1 to 9, wherein
v poly(ethylene oxide) homopolymer comprises terminal hydroxyl groups.

11. Composition according to any of claims 1 to 10, wherein the composition comprises iv 0,5 wt. % to 5 wt. % of an ammonium or alkali salt of a copolymer of acryloyldimethyltaurate and vinylpyrrolidone.

12. Composition according to any of claims 1 to 11, wherein
iii is a poly(ethylene oxide)-poly(propylene oxide)- poly(ethylene oxide) block copolymer, in particular with a medium molecular weight of 9840 to 14600 Dalton (Dalton D = unit for the relative molecular mass).

13. Use of a salt of a co-polymer comprising a copolymer of an ammonium or alkali salt of acryloyldimethyltaurate and vinylpyrrolidone, for encapsulation of a mixture comprising at least hydrogen peroxide, water and poly(ethylene oxide) homopolymer, or for forming a matrix comprising a mixture of at least hydrogen peroxide, water and poly(ethylene oxide) homopolymer in a composition according to any of claims 1 to 12.

14. Use of the composition according to any of claims 1 to 12, as oral care application, in particular tooth bleaching composition/tooth whitening composition.

## Patentansprüche

1. Zusammensetzung umfassend
i 2 Gew.-% bis 30 Gew.-% Wasserstoffperoxid,
ii Wasser,
iii 1 Gew.-% bis 10 Gew.-% Polyethylenoxid-Polypropylenoxid-Blockcopolymer,
iv 0,01 Gew.-% bis 10 Gew.-% eines Salzes eines Copolymers umfassend ein Copolymer eines Ammonium- oder Alkali-Salzes von Acryloyldimethyltaurat und Vinylpyrrolidon,
v 30 Gew.-% bis 95 Gew.-% Polyethylenoxid-Homopolymer,
vi 0,01 Gew.-% bis 5 Gew.-% Stabilisatorsystem ausgewählt aus
a) Phosphor-enthaltendem Stabilisator oder Mischung aus Phosphor-enthaltenden Stabilisatoren ausgewählt aus der Gruppe bestehend aus Pyrophosphaten, und
b) Stannat-Stabilisator, und/oder c) Alkalihydroxid, Base und/oder Puffer, und
vii 0,01 Gew.-% bis 1 Gew.-% eines Komplexbildners oder einer Mischung von Komplexbildnern, wobei die Zusammensetzung insgesamt 100 Gew.-% umfasst.

2. Zusammensetzung gemäß Anspruch 1, umfassend
i 5 Gew.-% bis 15 Gew.-% Wasserstoffperoxid,
ii 5 Gew.-% bis 15 Gew.-% Wasser,
iii 2 Gew.-% bis 10 Gew.-% Polyethylenoxid-Polypropylenoxid-Blockcopolymer,
iv 0,01 Gew.-% bis 10 Gew.-% eines Salzes eines Copolymers umfassend ein Copolymer eines Ammonium- oder Alkali-Salzes von Acryloyldimethyltaurat und Vinylpyrrolidon,
v 60 Gew.-% bis 85 Gew.-% Polyethylenoxid-Homopolymer,
vi 0,01 Gew.-% bis 5 Gew.-% Stabilisatorsystem ausgewählt aus
a) Phosphor-enthaltendem Stabilisator oder Mischung aus Phosphor-enthaltenden Stabilisatoren ausgewählt aus der Gruppe bestehend aus Pyrophosphaten, und
b) Stannat-Stabilisator, und/oder c) Alkalihydroxid, Base und/oder Puffer, und
vii 0,01 Gew.-% bis 1 Gew.-% eines Komplexbildners oder einer Mischung von Komplexbildnern, wobei die Zusammensetzung insgesamt 100 Gew.-% umfasst.

3. Zusammensetzung gemäß Anspruch 1, umfassend
i 15 Gew.-% bis 30 Gew.-% Wasserstoffperoxid,
ii 15 Gew.-% bis 30 Gew.-% Wasser,
iii 2 Gew.-% bis 10 Gew.-% Polyethylenoxid-Polypropylenoxid-Blockcopolymer,
iv 0,01 Gew.-% bis 10 Gew.-% eines Salzes eines Copolymers umfassend ein Copolymer eines Ammonium- oder Alkali-Salzes von Acryloyldimethyltaurat und Vinylpyrrolidon,
v 30 Gew.-% bis 60 Gew.-% Polyethylenoxid-Homopolymer,
vi 0,01 Gew.-% bis 5 Gew.-% Stabilisatorsystem ausgewählt aus
a) Phosphor-enthaltendem Stabilisator oder Mischung aus Phosphor-enthaltenden Stabilisatoren ausgewählt aus der Gruppe bestehend aus Pyrophosphaten, und
b) Stannat-Stabilisator, und/oder c) Alkalihydroxid, Base und/oder Puffer, und
vii 0,01 Gew.-% bis 1 Gew.-% eines Komplexbildners oder einer Mischung von Komplexbildnern, wobei die Zusammensetzung insgesamt 100 Gew.-% umfasst.

4. Zusammensetzung nach einem der Ansprüche 1 bis 3, wobei der Gewichtsanteil (Gew.-%) von Wasserstoffperoxid und Wasser von 2 : 1 bis 1 : 2 ist, insbesondere 1 : 1 +/-10%.

5. Zusammensetzung nach einem der Ansprüche 1 bis 4, wobei
iv das Copolymer ausgewählt ist aus einem Copolymer eines Ammoniums von Acryloyldimethyltaurat und Vinylpyrrolidon.

6. Zusammensetzung nach einem der Ansprüche 1 bis 3, wobei
vii der Komplexbildner oder die Mischung von Komplexbildnern ausgewählt ist aus Ethylendiamintetraessigsäure, Alkali- und/oder Erdalkalisalz von Ethylendiamintetraessigsäure (EDTA), aromatischem Komplexbildner und einer Mischung dieser, insbesondere Alkali- und/oder Erdalkalisalz von Ethylendiamintetraessigsäure (EDTA), besonders bevorzug ist Kalium- und/oder Natrium-ETDA.

7. Zusammensetzung nach einem der Ansprüche 1 bis 3, wobei
vi Pyrophosphat ausgewählt ist aus Diethylentriamin-penta(methylenphosphonsäure), Hexamethylendiamin-tetra(methylenphosphonsäure), Bis(hexamethylentriaminpenta(methylenphosphonsäure)) und Etidronsäure.

8. Zusammensetzung nach einem der Ansprüche 1 bis 3, wobei
vi der b) Stannat-Stabilisator Natriumstannat-Trihydrat ist.

9. Zusammensetzung nach einem der Ansprüche 1 bis 7, wobei die Zusammensetzung umfasst v 35 Gew.-% bis 85 Gew.-% Polyethylenoxid-Homopolymer.

10. Zusammensetzung nach einem der Ansprüche 1 bis 9, wobei
v Polyethylenoxid-Homopolymer terminale Hydroxylgruppen umfasst.

11. Zusammensetzung nach einem der Ansprüche 1 bis 10, wobei die Zusammensetzung umfasst
iv 0,5 Gew.-% bis 5 Gew.-% eines Ammonium- oder Alkalisalzes eines Copolymers von Acryloyldimethyltaurat und Vinylpyrrolidon.

12. Zusammensetzung nach einem der Ansprüche 1 bis 11, wobei
iii ein Polyethylenoxid-Polypropylenoxid-Polyethylenoxid-Blockcopolymer ist, insbesondere mit einem mittleren Molekulargewicht von 9840 bis 14600 Dalton (Dalton D = Einheit der relativen Molekülmasse).

13. Verwendung eines Salze seines Copolymers umfassend ein Copolymer eines Ammonium- oder Alkalisalzes von Acryloyldimethyltaurat und Vinylpyrrolidon, zur Verkapselung einer Mischung umfassend zumindest Wasserstoffperoxid, Wasser und Polyethylenoxid-Homopolymer, oder zur Ausbildung einer Matrix umfassend eine Mischung aus zumindest Wasserstoffperoxid, Wasser und Polyethylenoxid-Homopolymer in einer Zusammensetzung nach einem der Ansprüche 1 bis 12.

14. Verwendung der Zusammensetzung nach einem der Ansprüche 1 bis 12, als Mundpflege-Anwendung, insbesondere Zahnaufhellungszusammensetzung/ Zahnweißungszusammensetzung.

## Revendications

1. Une composition comprenant
i 2 % en poids à 30 % en poids du peroxyde d'hydrogène,
ii de l'eau,
iii 1 % en poids à 10 % en poids du copolymère en bloc d'oxyde de polyéthylène - d'oxyde de polypropylène,
iv 0,01 % en poids à 10 % en poids d'un sel d'un copolymère comprenant un copolymère d'un sel d'ammonium ou alcalin du taurate de diméthyle d'acryloyle et du pyrrolidone de vinyle,
v 30 % en poids à 95 % en poids de l'homopolymère d'oxyde de polyéthylène,
vi 0,01 % en poids à 5 % en poids du système de stabilisateurs sélectionné parmi du
a) stabilisateur contenant du phosphore ou de la mixture des stabilisateurs contenant du phosphore sélectionné(e) parmi du groupe consistant en pyrophosphates, et
b) du stabilisateur de stannate, et/ou c) de l'hydroxyde alcalin, de la base et/ou des tampons, et
vii 0,01 % en poids à 1 % en poids d'un agent de chélation ou d'une mixture des agents de chélation, selon laquelle la composition comprend au total 100 % en poids.

2. Composition selon la revendication 1, comprenant
i 5 % en poids à 15 % en poids du peroxyde d'hydrogène,
ii 5 % en poids à 15 % en poids de l'eau,
iii 2 % en poids à 10 % en poids du copolymère en bloc d'oxyde de polyéthylène - d'oxyde de polypropylène,
iv 0,01 % en poids à 10 % en poids du sel d'un copolymère comprenant un copolymère d'un sel d'ammonium ou alcalin du taurate de diméthyle d'acryloyle et du pyrrolidone de vinyle,
v 60 % en poids à 85 % en poids de l'homopolymère d'oxyde de polyéthylène,
vi 0,01 % en poids à 5 % en poids du système de stabilisateurs sélectionné parmi du a) stabilisateur contenant du phosphore ou de la mixture des stabilisateurs contenant du phosphore sélectionné(e) parmi du groupe consistant en pyrophosphates, et b) du stabilisateur de stannate, et/ou c) de l'hydroxyde alcalin, de la base et/ou des tampons, et
vii 0,01 % en poids à 1 % en poids d'un agent de chélation ou d'une mixture des agents de chélation, selon laquelle la composition comprend au total 100 % en poids.

3. Composition selon la revendication 1, comprenant
i 15 % en poids à 30 % en poids du peroxyde d'hydrogène,
ii 15 % en poids à 30 % en poids de l'eau,
iii 2 % en poids à 10 % en poids du copolymère en bloc d'oxyde de polyéthylène - d'oxyde de polypropylène,
iv 0,01 % en poids à 10 % en poids du sel d'un copolymère comprenant un copolymère d'un sel d'ammonium ou alcalin du taurate de diméthyle d'acryloyle et du pyrrolidone de vinyle,
v 30 % en poids à 60 % en poids de l'homopolymère d'oxyde de polyéthylène,
vi 0,01 % en poids à 5 % en poids du système de stabilisateurs sélectionné parmi du a) stabilisateur contenant du phosphore ou de la mixture des stabilisateurs contenant du phosphore sélectionné(e) parmi du groupe consistant en pyrophosphates, et b) du stabilisateur de stannate, et/ou c) de l'hydroxyde alcalin, de la base et/ou des tampons, et
vii 0,01 % en poids à 1 % en poids d'un agent de chélation ou d'une mixture des agents de chélation, selon laquelle la composition comprend au total 100 % en poids.

4. Composition selon l'une des revendications 1 à 3, selon laquelle le teneur de poids (% en poids) du peroxyde d'hydrogène et de l'eau sont de 2 : 1 à 1 : 2, en particulier 1 : 1 +/-10%.

5. Composition selon l'une des revendications 1 à 4, selon laquelle
iv le copolymère est sélectionné parmi d'un copolymère d'un ammonium du taurate de diméthyle d'acryloyle et du pyrrolidone de vinyle.

6. Composition selon l'une des revendications 1 à 3, selon laquelle
vii l'agent de chélation ou la mixture des agents de chélation, est sélectionné(e) parmi de l'acide éthylènediamine tétraacétique, du sel alcalin et/ou alcalino-terreux de l'acide éthylènediamine tétraacétique (EDTA), de l'agent de chélation aromatique et une mixture de tels, en particulier du sel alcalin et/ou alcalino-terreux de l'acide éthylènediamine tétraacétique (EDTA), préférés particulièrement sont l'ÉDTA de potassium et/ou sodium.

7. Composition selon l'une des revendications 1 à 3, selon laquelle
vi le pyrophosphate est sélectionné parmi de l'acide diéthylènetriamine-pentaméthylène phosphonique, de l'acide hexaméthylènediamine-tetraméthylène phosphonique, de l'acide bis(hexaméthylènetriamine-pentaméthylène phosphonique) et de l'acide étidronique.

8. Composition selon l'une des revendications 1 à 3, selon laquelle
vi le b) stabilisateur de stannate est le stannate de sodium trihydrate.

9. Composition selon l'une des revendications 1 à 7, selon laquelle la composition comprend v 35 % en poids à 85 % en poids du homopolymère d'oxyde de polyéthylène.

10. Composition selon l'une des revendications 1 à 9, selon laquelle
v l'homopolymère d'oxyde de polyéthylène comprend des groupes hydroxyles
terminales.

11. Composition selon l'une des revendications 1 à 10, selon laquelle la composition comprend
iv 0,5 % en poids à 5 % en poids d'un sel d'ammonium ou alcalin d'un copolymère du taurate de diméthyle d'acryloyle et du pyrrolidone de vinyle.

12. Composition selon l'une des revendications 1 à 11, selon laquelle
iii est un copolymère en bloc d'oxyde de polyéthylène - d'oxyde de polypropylène - d'oxyde de polyéthylène, en particulier avec un poids moléculaire moyen de 9840 à 14600 Dalton (Dalton D = l'unité de masse moléculaire relative).

13. Utilisation d'un sel d'un copolymère comprenant un copolymère d'un sel d'ammonium ou alcalin du taurate de diméthyle d'acryloyle et du pyrrolidone de vinyle, pour l'encapsulation d'une mixture comprenant au moins un peroxyde d'hydrogène, l'eau et de l'homopolymère d'oxyde de polyéthylène, ou pour former une matrice comprenant une mixture d'au moins une peroxyde d'hydrogène, l'eau et de l'homopolymère d'oxyde de polyéthylène dans un composition selon l'une des revendications 1 à 12.

14. Utilisation de la composition selon l'une des revendications 1 à 12, en tant qu'une application d'hygiène buccale, en particulier une composition blanchissant des dents/composition de blanchiment des dents.
